# EUROPEAN PATENT SPECIFICATION

(11) **EP 3 518 246 B1**
(45) Date of publication and mention of the grant of the patent: **18.11.2020**
(21) Application number: 18211580.8
(22) Date of filing: 12.04.2013
(51) Int. Cl.: G16H 40/63, G06F 3/048, G06F 13/38

(54) **SYSTEMS AND METHODS FOR COLLECTING AND VIEWING PATIENT DATA**
SYSTEME UND VERFAHREN ZUM ERFASSEN UND ANZEIGEN VON PATIENTENDATEN
SYSTÈMES ET MÉTHODES DE COLLECTE ET DE VISUALISATION DES DONNÉES D'UN PATIENT

(30) Priority: 16.04.2012 US 201261624951 P
(43) Date of publication of application: 31.07.2019
(62) Divisional of application: 13777642.3
(73) Proprietor: AirStrip IP Holdings, LLC, San Antonio TX 78258 (US)
(72) Inventor: MOORE, Stephen Trey, San Antonio, TX Texas 78261 (US); WADE, Thomas Scott, Wimberley, TX Texas 78676 (US); BROWN, Lloyd Kory, Georgetown, TX Texas 78626 (US)
(74) Representative: Fish & Richardson P.C.

(56) References cited:
- WO-A2-2006/111874
- US-A1- 2009 005 651
- US-A1- 2012 054 401
- US-A1- 2012 075 103

## Description

### BACKGROUND

Implementations of the present disclosure are directed to displaying patient data and/or information on mobile devices.

While physicians and other health care providers currently utilize a large number of products and systems that benefit from advances in wireless communication technology, there are still significant limitations to the information that can be transmitted, received, and displayed over these devices in a practical and efficient manner. There are many limitations that are intrinsic to mobile devices, especially those constraints related to speed, performance, memory, and display size. In addition, because of the critical nature of medical data, it is important that the technology work reliably and efficiently over potentially low speed, low bandwidth, and sometimes intermittent wireless connections. US 2012/0075103 describes methods of measuring features of a digitally generated waveform. The methods include communicating patient data to a device that is remote from a source of the patient data, generating the waveform on a touch-screen display of the device, and measuring along an axis of the waveform.
WO 2006/111874 describes an ultrasonic diagnostic imaging system that is operable in the docked mode or in the portable mode. In the undocked mode a portable ultrasound system is operated by means of its own user interface and flat panel display. In the docked mode the portable ultrasound system is docked with a docking station and the flat panel display of the portable system provides the docked system display. The docking station resembles a typical cart-borne ultrasound system with a base unit and a control panel. The portable ultrasound system provides the display of the cart-borne system when docked.
US 2009/0005651 describes a portable monitor device that includes an integral display to act portably to display various information. The portable monitor device is configured for placement selectively in a docking station.
US 2012/0054401 describes a docking device that includes a screen, controls, and a receptacle for holding a mobile device. The mobile device may generate and display information for on its screen while concurrently generating and outputting information to the docking device for display on the screen of the docking device.

### SUMMARY

The invention is defined in the attached claims. The present patent generally describes methods that include the actions of receiving, by a tablet computing device, one or more signals reflective of patient data associated with one or more patients, the tablet computing device being provided in a patient monitoring system that monitors physiological characteristics of a patient of the one or more patients, at least a first sub-set of the patient data being associated with the patient and being provided from one or more sensors that are responsive to the physiological characteristics of the patient, processing the patient data to generate one or more graphical representations reflective of the patient data, and displaying the one or more graphical representations on a touchscreen display of the tablet computing device.

In some related methods, the one or more sensors are in direct communication with the tablet computing device within the patient monitoring system.

In some related methods, direct communication includes at least one of wired communication and wireless communication.

In some related methods, a second sub-set of the patient data is provided from an information system associated with a facility within which the one or more patients are treated.

In some related methods, a third sub-set of the patient data is provided from a data management system (DMS).

In some related methods, the one or more graphical representations include waveforms based on the first sub-set of patient data.

In some related methods, the one or more graphical representations comprise waveforms associated with physiological characteristics of a plurality of patients of the one or more patients.

According to the present disclosure, actions include determining that the tablet computing device is docked in a docking station of the patient monitoring system, and in response, executing the tablet computing device in a single application mode. When operating in the single application mode, the tablet computing device is only able to execute a patient monitoring application for receiving the one or more signals, processing the patient data and generating the one or more graphical representations. In addition, in response to determining that the tablet computing device is docked in the docking station, functionality of one or more buttons of the tablet computing device is disabled. In some examples, the one or more buttons include a home button and a sleep/wake button. In some examples, actions further include receiving user input including authentication data, determining that the user input corresponds to an authenticated user, and in response, exiting the single application mode such that the tablet computing device is able to execute a plurality of computer program applications.

Other aspects include one or more processors, and a computer-readable medium coupled to the one or more processors having instructions stored thereon which, when executed by the one or more processors, cause the one or more processors to perform one or more of the methods provided herein.

The details of one or more embodiments are set forth in the accompanying drawings and the description below. Other features, objects, and advantages will be apparent from the description and drawings, and from the claims.

### DESCRIPTION OF DRAWINGS

FIG. 1 is a schematic illustration of an example system architecture in accordance with the present disclosure.
FIG. 2 is a schematic illustration of another example system architecture in accordance with the present disclosure.
FIG. 3 is a schematic illustration of another example system architecture in accordance with the present disclosure.
FIG. 4 depicts example communication between components of the example system architecture of FIG. 3.
FIGs. 5-6B depict an example patient monitoring system for collecting and displaying patient data.

Like reference symbols in the various drawings indicate like elements.

### DETAILED DESCRIPTION

The present disclosure provides a healthcare provider with secure, remote access to patient data. For purposes of the instant description, and by way of non-limiting example, implementations of the present disclosure will be described in the context of patient data corresponding to maternity patients (e.g., obstetric (OB) patient). Implementations of the present disclosure are applicable to any variety of patients and corresponding patient data.

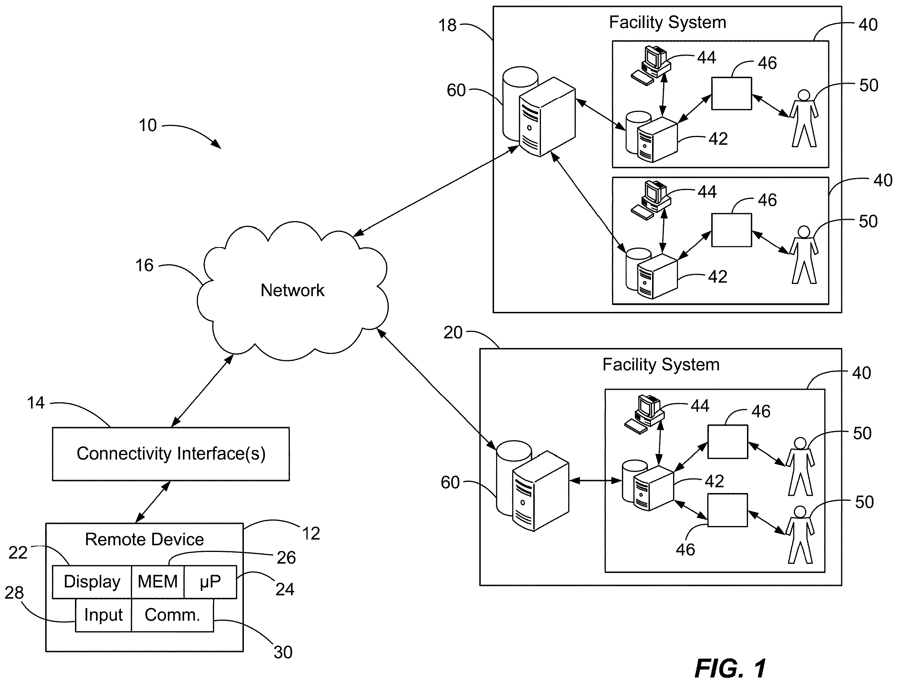

Referring now to FIG. 1, an example system architecture 10 is illustrated, and includes a remote device 12, connectivity interface(s) 14, a network 16, a first facility system 18, and a second facility system 20. As discussed in further detail herein, data is transferred from each of the first and second facility systems 18, 20 through the network 16 and connectivity interface(s) 14 for presentation, or display on the remote device 12. Further, data can be transferred from the remote device 12 through the connectivity interface(s) 14 and network 16 to each of the first and second facility systems 18, 20. Although a single remote device 12 is illustrated, it is contemplated that one or more remote devices 12 can communicate with each of the first and second facility systems 18, 20 through the network 16 and connectivity interface(s) 14. Similarly, although two facility systems are illustrated, the present disclosure can be implemented with one or more facility systems.

The remote device 12 can include any number of example devices. Such example devices include, but are not limited to, a mobile phone, a smartphone, a personal digital assistant (PDA), a laptop, a tablet personal computer (PC), a desktop PC, and/or combinations thereof. The remote device 12 includes a display 22, a processor 24, memory 26, an input interface 28, and a communication interface 30. The processor 24 can process instructions for execution of implementations of the present disclosure. The instructions can include, but are not limited to, instructions stored in the memory 26 to display graphical information on the display 22. Example displays include, but are not limited to, a thin-film-transistor (TFT) liquid crystal display (LCD), or an organic light emitting diode (OLED) display.

The memory 26 stores information within the remote device 12. In some implementations, the memory 26 can include a volatile memory unit or units, and/or a non-volatile memory unit or units. In other implementations, removable memory can be provided, and can include, but is not limited to, a memory card. Example memory cards can include, but are not limited to, a secure digital (SD) memory card, a mini-SD memory card, a USB stick, and the like.

The input interface 28 can include, but is not limited to, a keyboard, a touchscreen, a mouse, a trackball, a microphone, a touchpad, and/or combinations thereof. In some implementations, an audio codec (not shown) can be provided, which receives audible input from a user or other source through a microphone, and converts the audible input to usable digital information. The audio codec can generate audible sound, such as through a speaker that is provided with the remote device 12. Such sound may include, but is not limited to, sound from voice telephone calls, recorded sound (e.g., voice messages, music files, etc.), and sound generated by applications operating on the remote device 12.

The remote device 12 may communicate wirelessly through the communication interface(s) 14, which can include digital signal processing circuitry. The communication interface(s) 14 may provide communications under various modes or protocols including, but not limited to, GSM voice calls, SMS, EMS or MMS messaging, CDMA, TDMA, PDC, WCDMA, CDMA2000, and/or GPRS. Such communication may occur, for example, through a radio-frequency transceiver (not shown). Further, the remote device can be capable of short-range communication using features including, but not limited to, Bluetooth and/or WiFi transceivers (not shown).

The remote device 12 communicates with the network 16 through the connectivity interface(s) 14. The connectivity interface(s) 14 can include, but is not limited to, a satellite receiver, cellular network, a Bluetooth system, a Wi-Fi system (e.g., 802.x), a cable modem, a DSL/dial-up interface, and/or a private branch exchange (PBX) system. Each of these connectivity interfaces 14 enables data to be transmitted to/from the network 16. The network 16 can be provided as a local area network (LAN), a wide area network (WAN), a wireless LAN (WLAN), a metropolitan area network (MAN), a personal area network (PAN), the Internet, and/or combinations thereof.

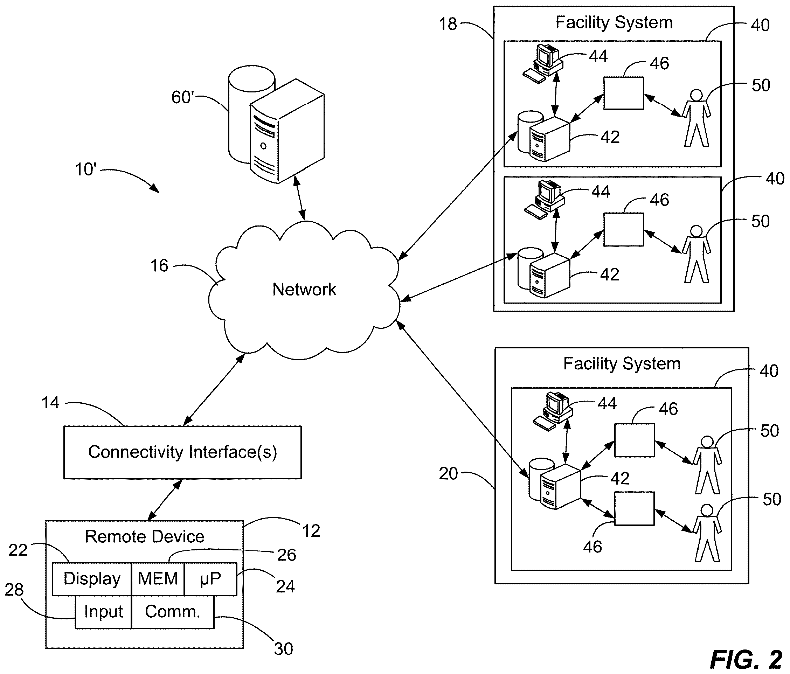

In the example systems of FIGs. 1 and 2, the first facility system 18 includes a plurality of facilities 40, and the second facility system 20 includes a facility 40. It is contemplated that each facility system 18, 20 can include one or more facilities, and is not limited to the example arrangement described herein. In the case of multiple facilities, the facilities can be remotely located from one another, and/or can be located at a common location, or site (e.g., separate departments in a common building). Each facility system 18, 20 can be provided as a medical care system, for example, which medical care system can include one or more hospitals, hospital systems, clinics, physician offices, and the like.

Each facility 40 includes an associated information system 42, computer interface(s) 44, and patient monitoring device(s) 46. Example information systems can include, but are not limited to, a clinical information system (CIS), and/or a hospital information system (HIS). Each information system 42 can be provided as a server, and supports the acquisition, storage, modification, and distribution of clinical information, such as patient data, throughout the facility 40 and/or facility system 18, 20. Each information system 42 can communicate with one or more ancillary information systems (not shown) that can include, but are not limited to, a pharmacy management system, a laboratory management system, and/or a radiology management system. Although the example system architecture 10 includes an information system 42 located at each facility 40, it is contemplated that the facilities 40 can communicate with a common information system 42 that is remotely located from either facility 40, or that is located at one of the facilities 40 within the facility system 18, 20.

The computer interface 44 can communicate with the information system 42 to enable access to information that is stored within, and managed by the information system 42. The computer interface 44 can include, but is not limited to, a personal computer (PC) (e.g., desktop, laptop, or tablet). Although a single computer interface 44 is illustrated in the example architectures described herein, it is contemplated that one or more computer interfaces 44 can communicate with the information system 42. Communication between each computer interface 44 and the information system 42 can be achieved via a direct connection, or remotely through a network (not shown) that can include, but is not limited to, a LAN, a WAN, a WLAN, and/or the Internet.

Each patient monitoring device 46 monitors physiological characteristics of a particular patient 50, and generates data signals based thereon. As discussed in further detail herein, implementations of the present disclosure provide patient monitoring devices that include a computing device, such as a tablet computing device. The data signals are communicated to the information system 42, which collects patient data based thereon, and stores the data to a patient profile that is associated with the particular patient. Although a single patient monitoring device 46 is illustrated per each patient 50, it is contemplated that multiple patient monitoring devices 46 can monitor a particular patient 50. The patient monitoring device(s) 46 can communicate with the information system 42 via a direct connection, or remotely through a network (not shown) that can include, but is not limited to, a LAN, a WAN, a WLAN, and/or the Internet.

The patient data is made available for display on the computer device 44. A healthcare provider (e.g., a nurse and/or physician) can augment the patient data by inputting patient information that is also stored to the information system 44. More specifically, the healthcare provider can input patient information corresponding to a particular patient 50, which patient information can be stored to the patient profile. By way of one non-limiting example, a nurse can input nursing notes, which nursing notes can be stored to the patient profile in the information system. As used herein, the term patient information includes any information corresponding to a patient that is input and stored to the information system 42 through the computer interface 44. Patient information is discussed in further detail below.

As discussed above, each information system 42 stores patient data that can be collected from the patient monitoring devices 46, as well as additional patient information, that can include information that is input by a healthcare provider. The information system 44 communicates the patient data and/or the additional patient data to a data management system (DMS) 60. The DMS 60 can be provided as a server, or a virtual server, that runs server software components, and can include data storage including, but not limited to, a database and/or flat files. In the example system architecture of FIG. 1, each facility system 18, 20 includes a corresponding DMS 60. In such an arrangement, each information system 42 communicates patient data, and/or additional patient data to the DMS 60. Furthermore, and as discussed in further detail below, the DMS 60 can communicate ancillary information to the information system 42. Communication between the DMS 60 and the information system(s) 42 can be achieved via a direct connection, or remotely through a network (not shown) that can include, but is not limited to, a LAN, a WAN, a WLAN, and/or the Internet.

A DMS 60 corresponding to a particular facility system can be remotely located from any of the facilities 40 of the facility system 18, 20, or can be located at a particular facility 40 of the facility system 18, 20. In the example system architecture of FIG. 1, the DMS 60 is remotely located from either facility 40 within each of the facility systems 18, 20. It is contemplated, however, that the DMS 60 can be located at one of the facilities 40, and remote from the other facility 40.

In the example system architecture of FIG. 2, a common DMS 60' is provided. The common DMS 60' is common to various facility systems 18, 20, and is not associated with a particular facility system 18, 20. Each information system 42 communicates with the DMS 60' via a direct connection, or remotely through a network (not shown) that can include, but is not limited to, a LAN, a WAN, a WLAN, and/or the Internet. In the example arrangement of FIG. 2, the DMS 60' communicates with each of the information systems 42 through the network 16. The information systems 42 communicate patient data and/or patient information to the DMS 60', and the DMS 60' can communicate ancillary information to the information system 42, as discussed in further detail below.

In the example system architecture of FIG. 1, the facility 40, or facility system 18, 20 installs the DMS 60 as a local DMS, and the DMS 60 sits at the local site with other servers that can include, but are not limited to, the information system 42. In some implementations, the DMS 60 can be sectioned off, or separated from a logical network perspective, but still physically exists with the other servers that belong to the respective facility 40. Server components are installed on the DMS 60, which components can include, but are not limited to, a database component, a database synchronization component, a web services component, and/or a structured query language (SQL) component. An information system interface can also be installed on the DMS 60, and functions as the interface to the information system 42. By way of non-limiting example, the information system interface can include OBLink, provided by GE Healthcare. In some implementations, the DMS 60 can be arranged in a multiple server configuration, in which one server only hosts web service related components and is logically segregated, and another server has the remaining necessary server components installed.

The example system architecture of FIG. 2, provides for the remote location of data collection at the DMS 60'. In such implementations, the DMS 60' can be provided at a third-party site, remote from any of the facilities 40, or facility systems 18, 20. The third-party functions as a DMS host, and the necessary server components are installed on the remotely hosted DMS 60'. In some implementations, a business-to-business (B2B) virtual private network (VPN) can be created between the remotely hosted DMS 60' and the network of the facility 40 or facility system 18, 20. In this manner, the facility 40 and/or facility system 18, 20 forgoes the purchase and/or maintenance of another physical server, or DMS 60. Further, the up-time and the status of availability of the DMS 60' are easier to manage on the part of a dedicated third-party. The DMS' access to the network can be attended to by the third-party, as opposed to burdening the facility 40, or the facility systems 18, 20. Further, the third-party can implement virtual server technologies to leverage multiple DMS installations on a single physical server. In such implementations, a plurality of virtual servers are logically partitioned in a single physical server, and each virtual server has the capability of running its own operating system and server components, and can be independently booted.

The DMS 60, 60' synchronizes and transfers data between the remote device 12, or multiple remote devices 12, and the information system 42, or multiple information systems 42. More specifically, the DMS 60, 60' processes and prepares the patient data and/or patient information for transfer to and presentation on the remote device 12, or multiple remote devices 12, from the information system 42. The DMS 60, 60' also processes and prepares ancillary information for transfer to and storage in the information system 42 from the remote device 12, or multiple remote devices 12 for potential presentation at a corresponding computer device 44. Example DMSs can include, but are not limited to, the AirStrip Server provided by AirStrip Technologies, LLC, which AirStrip Server includes AirStrip Server Components installed therein.

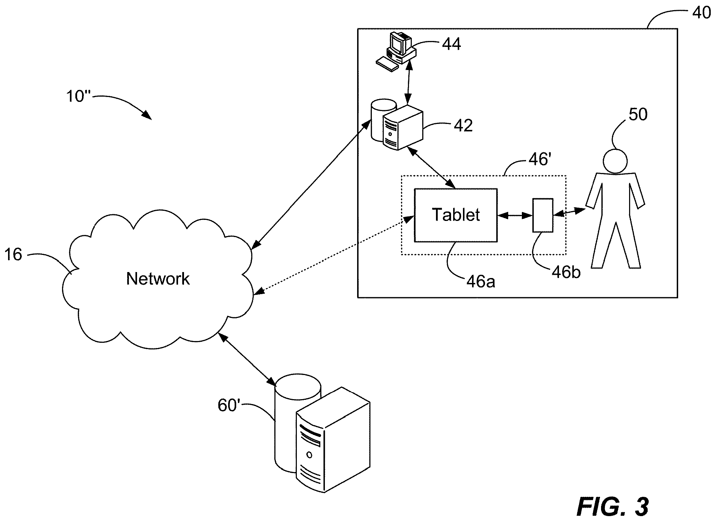

Referring now to FIG. 3 an example system architecture 10" is illustrated, and includes a facility system 40, a network 16, and a DMS 60'. As discussed in further detail herein, the facility system 40 includes one or more patient monitoring devices 46', an information system 42, and a computer interface 44.

Although a single patient monitoring device 46' is illustrated in the example architecture 10", it is contemplated that one or more patient monitoring device(s) 46' can be integrated in the system architecture. In some examples, patient monitoring device(s) 46' can be located within the facility system 40. In some examples, patient monitoring device(s) 46' can be located within and/or outside of the facility system 40. For example, a patient monitoring device 46' can be located at a patient's home (e.g., for in-home care). Communication between the patient monitoring device 46', the information system 42 and/or the DMS 60' can be achieved via a direct connection, and/or remotely through one or more networks (e.g., the network 16) that can include, but is not limited to, a LAN, a WAN, a WLAN, and/or the Internet.

In accordance with the present disclosure, the patient monitoring device 46' includes a tablet computing device 46a and one or more data collection systems 46b. In some examples, the tablet computing device 46a can execute one or more computer program applications to display patient data waveforms (patient waveforms) based on data received from the one or more data collection systems 46b. In some examples, the data collection systems 46b include one or more peripheral devices (e.g., sensors) that are responsive to physiological characteristics of a patient (e.g., the patient 50). Example peripheral devices can include, but are not limited, a blood pressure monitoring device, a heart rate monitoring device, a fetal heart rate monitoring device, a contraction monitoring device, an electrocardiograms (ECG) device, and/or any other appropriate device that is responsive the physiological characteristics of a patient and that generates data signals based thereon.

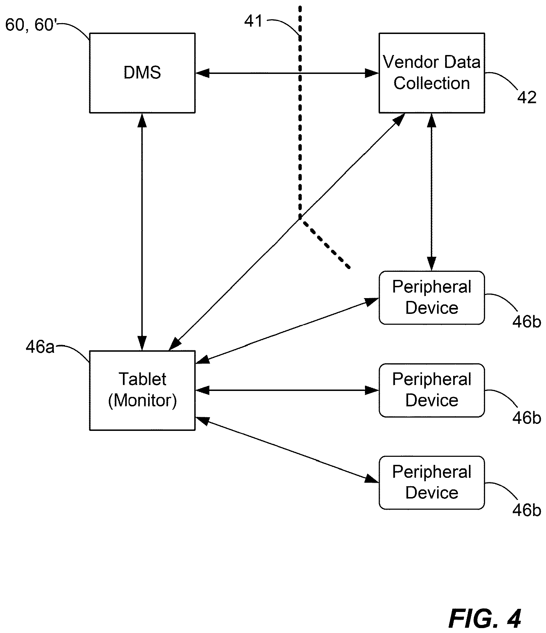

FIG. 4 depicts an example sub-architecture that indicates example communication paths between various components (e.g., provided in FIG. 3). In the depicted example, a tablet computing device 46a (operating as a patient monitoring device) communicates with one or more of a DMS 60, 60', an information system 42 (identified as a vendor data collection system) and one or more peripheral devices 46b. In some implementations, the tablet computing device 46a communicates with the DMS 60, 60' and/or the information system 42 over one or more networks (e.g., the network 16). In some examples, the tablet computing device 46a communicates with the one or more peripheral devices 46b over one or more networks (e.g., the network 16), directly (e.g., a wired connection) and/or wirelessly (e.g., Bluetooth® connection). In some examples, one or more of the peripheral devices 46b can communicate with the information system 42 over one or more networks (e.g., the network 16). In some examples, communication with the information system 42 can occur through a gateway 41. In this manner, devices communicating with the information system 41 can be authenticated prior to data transfer between the information system 42 and the devices.

In some implementations, the tablet computing device 46a receives patient data from the one or more peripheral devices 46b. The tablet computing device 46a processes the patient data and displays graphical representations of the patient data. Example graphical representations can include patient waveforms, textual data, alarms and/or indicators. In some examples, the tablet computing device 46a provides the patient data received from the one or peripheral devices to one or more of the DMS 60, 60' and the information system 42. In some examples, the tablet computing device 46a provides the patient data to the information system 42 and the information system 42 provides the patient data to the DMS 60, 60' (as discussed above). In some implementations, one or more of the peripheral devices 46b provides the patient data to the information system 42 and the information system 42 can provide the patient data to the tablet computing device 46a and/or the DMS 60, 60'. In some examples, the DMS provides data (e.g., ancillary data) to the tablet computing device 46a and/or the information system 42.

The tablet computing device 46a receives physiological data of a particular patient 50 and monitors the physiological characteristics of the particular patient. In some implementations, each tablet computing device 46a receives physiological data of multiple particular patients 50 and monitors the physiological characteristics of the multiple patients. In this manner, for example, a healthcare provider can review patient data for any one of multiple patients from any location. For example, the tablet computing device 46a can be located within a room of a first patient and can be receiving patient data associated with the first patient from one or more peripheral devices 46b. A healthcare provider can access the tablet computing device 46a in the room of the first patient to retrieve and display patient data associated with a second patient (e.g., retrieve the patient data associated with the second patient from the information system 42).

In some implementations, the tablet computing device 46a executes one or more computer programs to provide a variety of services. Example services can include information display, charting, querying, retrieving and displaying electronic medical records (EMRs), reviewing and interacting with historical waveforms, image generation and/or teleconferencing (*e.g*. from caregiver to caregiver and/or caregiver to patient).

The tablet computing device 46a includes a touchscreen display that enables user interaction with the tablet computing device 46a. For example, a user (e.g., a healthcare provider) can interact with the touchscreen display to request the display of patient data, waveforms, digital images, digital video, EMRs and the like. In some examples, and as discussed in further detail below with regard to FIGs. 9A-9J, the user can perform historical scrolling of patient waveform data using the touchscreen display. The historical scrolling of patient waveform data can be bidirectional, enabling the user to scroll through older or more recent medical data and chart data at specified intervals.

In some implementations, the tablet computing device 46a can include a camera and software that enables acquisition of digital images and/or digital video. In some examples, the tablet computing device 46a can include a microphone for capturing audio (e.g., dictated notes). In this manner, a healthcare provider can use the tablet computing device 46a to generate digital images, digital video and/or digital audio associated with the patient (e.g., images tracking wound care). One or more electronic files including the digital image, digital video and/or digital audio can be provided to one or more of the information system 42 and the DMS 60, 60'. In some examples, the electronic files can be appended to, or otherwise become part of an EMR associated with the patient. In some examples, a tele- and/or video-conference can be established between a user of the tablet computing device 46a and a user of another, remote computing device. For example, a first healthcare provider (e.g., a nurse) can use the tablet computing device 46a and a second healthcare provider (e.g., a doctor) can use a remote device (e.g., the remote device 12, discussed above). A tele- and/or video-conference can be established between the first healthcare provider and the second healthcare provider using the tablet computing device 46a and the remote device.

In some implementations, the digital imaging, digital videoing and/or conferencing (tele/video) functionality can be provided by a patient monitoring application executed on the tablet computing device 46a. In this manner, for example, resultant data (e.g., digital image files, digital video files, digital audio files) can be automatically associated with one or more patients, can be appended to respective EMRs associated with the one or more patients, and/or can be automatically transferred to one or more of the information system 42 and the DMS 60, 60'.

In some implementations, if a physiological parameter exceeds a predetermined range (e.g. blood pressure increases or decreases significantly, a cardiac event occurs and/or oxygen saturation drops), a visual and/or audible alarm can be activated. In some examples, the tablet computing device 46a can generate the visual and/or audible alarm. In some implementations, the tablet computing device 46a can be used to dismiss the alarm.

The tablet computing device 46a is configured to operate in a single application mode. In the single application mode, the tablet computing device 46a executes a single application (e.g., a patient monitoring application) and prevents execution of other applications. For example, the tablet computing device 46a can be executing a patient monitoring application as part of a patient monitoring device 46' for a particular patient. With the tablet computing device 46a executing in the single application, the patient and/or visitors visiting the patient are prevented from accessing the tablet computing device 46a to execute other applications on the tablet computing device. In this manner, the tablet computing device 46a is set to display only the medical data acquired from the patient, or multiple patients.

According to the present disclosure, functionality of the tablet computing device 46a is locked out. For example, functionality that would normally execute in response to user input (e.g., user contact with the touchscreen display, user contact with one or more buttons) is prevented from execution in response to user input. In some examples, one or more application program interfaces (APIs) associated with input devices (e.g., the touchscreen, buttons (home button, sleep/wake button) can be accessed to instruct prevent functionality from executing in response to user input. In this manner, the tablet computing device 46a can execute as instructed (e.g., as a patient monitor) without interruption.

In some implementations, the single application mode and/or the functionality lockout can be overridden. For example, an authorized user can provide credentials (e.g., username and password) to override the single application mode and/or the functionality lockout.

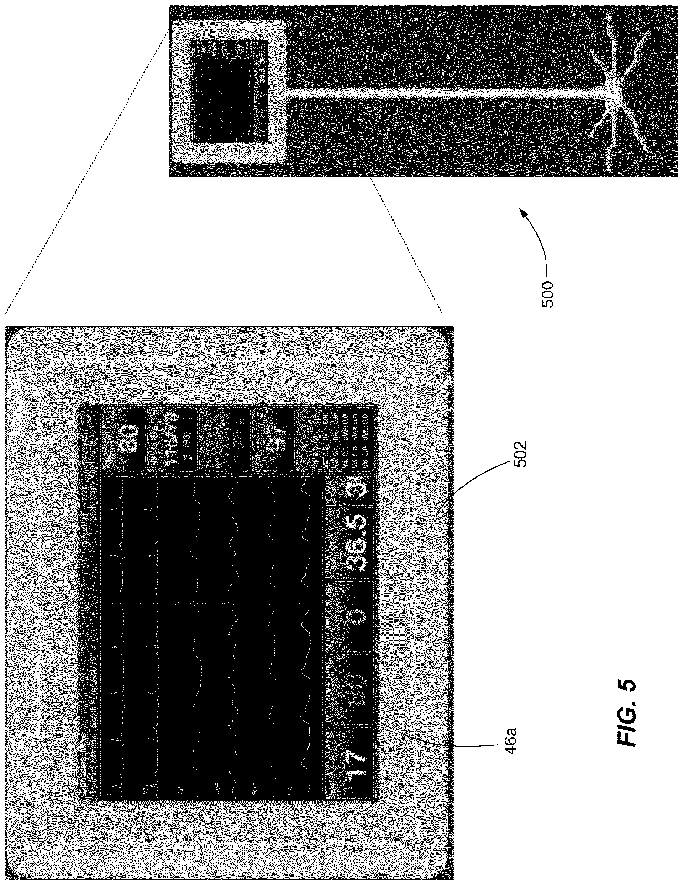

FIG. 5 illustrates an example monitoring system 500 (e.g., patient monitoring device 46'). The monitoring system 500 includes the tablet computing device 46a and a docking station 502. For example, the tablet computing device 46a can be securely docked with the docking station 502. In some example, the docking station 502 is mounted on a mobile stand, allowing facile displacement. In the depicted example, the tablet computing device 46a is displaying fictitious patient data associated with a fictitious patient, the patient data reflecting physiological data associated with cardiovascular characteristics of the fictitious patient. It is appreciated, however, that other physiological data can also be retrieved, monitored and displayed by the tablet computing device 46a.

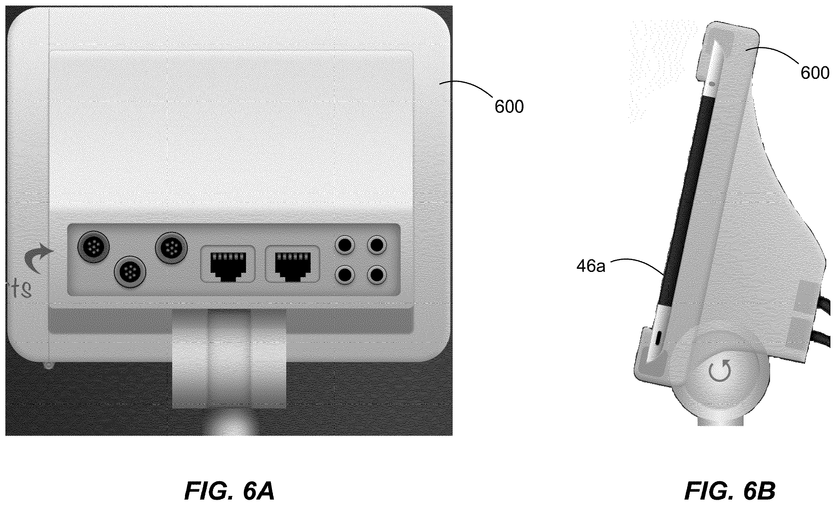

FIG. 6A and 6B provide a detailed representation of an example docking station 600. In some implementations, as seen in FIG. 6B, the docking station 600 can include multiple ports for wired connection with peripheral devices (e.g., the peripheral devices 46b). In some examples, the ports are compatible with standard medical devices to acquire physiological data (*e.g.* ECG signals, blood pressure waveforms, heart rate, etc.). As depicted in FIG. 6B, the tablet computing device 46a can be docked with the docking station 600. In the depicted example, the tablet computing device 46a slides into a slot that is arranged to receive the tablet computing device 46a. In some examples, the tablet computing device 46a can be locked into the docking station 600 to prevent removal of the tablet computing device 46a. For example, a locking mechanism can be provided and can require a key or other unlocking means (e.g., code) to release the tablet computing device 46a from the docking station 600.

In some examples, the tablet computing device 46a can be in electrical communication with the docking station 46a. In some examples, the docking station 600 receives electrical signals from the peripheral devices and can provide the signals to the tablet computing device in real-time. In some examples, electrical communication between the tablet computing device and the docking station can be achieved via wired communication and/or wireless communication (e.g., WiFi, Bluetooth ®). In some implementations, the docking station 600 can be plugged into an electrical source (e.g., an outlet) and can charge the battery of the tablet computing device 46a. In some implementations, as illustrated by FIG. 6B, the docking station 600 can be tilted at different angles, enabling the user to choose the most convenient angle for viewing and/or interacting with the tablet computing device 46a (monitor).

According to the present disclosure, communication between the tablet computing device 46a and the docking station 600 can trigger operation of the tablet computing device 46a in the single application mode. In response to the tablet computing device 46a being docked into the docking station 600, the single application mode is automatically triggered. In some examples, in response to the tablet computing device 46a establishing communication with the docking station 600, the single application mode can be automatically triggered.

Implementations of the present disclosure can be provided using digital electronic circuitry, or in computer hardware, firmware, software, or in combinations thereof. In some examples, implementations can be provided one or more computer program products, e.g., a computer program tangibly embodied in a machine-readable storage device, for execution by, or to control the operation of, data processing apparatus, and/or a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a communication network. Such a computer program can include modules and/or code segments for executing one or more of the features, aspects and/or implementations provided herein.

Operations in accordance with implementations of the present disclosure can be performed by one or more programmable processors executing a computer program product to perform functions by operating on input data and generating output. By way of example, a computer program product can include modules and/or code segments corresponding to each of the method steps, aspects and/or features provided herein. Method steps can also be performed by, and apparatus of the present disclosure can be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. Elements of a computer can include a processor for executing instructions and one or more memory devices for storing instructions and data. Generally, a computer can also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, e.g., EPROM, EEPROM, and flash memory devices; magnetic disks such as internal hard disks and removable disks; magneto-optical disks; and CD-ROM and DVD-ROM disks. The processor and the memory can be supplemented by, or incorporated in special purpose logic circuitry.

The present disclosure can be implemented in a system including, but not limited to the example systems described herein, which include a back-end component, e.g., as a data server, or that includes a middleware component, e.g., an application server, or that includes a front-end component, e.g., a client device, such as the mobile device 102, having a graphical user interface or a Web browser through which a user can interact with an implementation of the invention, or any combination of such back-end, middleware, or front-end components. The components of the system can be interconnected by any form or medium of digital data communication, e.g., a communication network.

## Claims

1. A computer-implemented method for providing a user of a tablet computing device (46a) access to patient data, executed using one or more processors, the method comprising:
determining that the tablet computing device (46a) is docked in a docking station (600) of a patient monitoring system (500); and
in response to determining that the tablet computing device (46a) is docked in a docking station (600) of the patient monitoring system (500), automatically triggering execution, by the tablet computing device (46a), of a single application, which is a patient monitoring application, preventing execution of other applications, and disabling functionality of one or more buttons of the tablet computing device (46a); receiving, by the tablet computing device (46a) from a data management system (DMS) (60, 60') configured to retrieve the patient data from a plurality of remotely located facility systems (18, 20, 40), the patient data comprising one or more signals associated with one or more patients(50), at least a first sub-set of the patient data being associated with the patient (50) and being provided from one or more sensors that are responsive to the physiological characteristics of the patient (50);
processing the patient data to generate one or more graphical representations reflective of the patient data; and
displaying the one or more graphical representations on a touchscreen display of the tablet computing device (46a).

2. The method of claim 1, wherein the DMS (60, 60') synchronizes and transfers data between a plurality of remote devices (12), and one or more information systems (42).

3. The method of claim 2, wherein the DMS (60, 60') processes and prepares ancillary information for transfer to and storage in the information system 42 from the plurality of remote devices (12).

4. The method of claim 1, wherein a second sub-set of the patient data is provided from an information system associated with a facility within which the one or more patients (50) are treated.

5. The method of claim 1, wherein the one or more graphical representations comprise waveforms based on the first sub-set of patient data.

6. The method of claim 1, wherein the one or more graphical representations comprise waveforms associated with physiological characteristics of a plurality of patients (50) of the one or more patients (50).

7. The method of claim 1, wherein the one or more buttons comprise a home button and a sleep/wake button.

8. The method of claim 1, further comprising:
receiving user input comprising authentication data;
determining that the user input corresponds to an authenticated user; and
in response, exiting the single application mode such that the tablet computing device (46a) is able to execute a plurality of computer program applications.

9. The method of claim 1, wherein the docking station (600) further comprises one or more ports compatible with standard medical devices.

10. The method of claim 1, wherein the docking station (600) comprises a slot that is configured to receive the tablet computing device (46a).

11. The method of claim 1, wherein the docking station (600) comprises a locking mechanism that is configured to prevent removal of the tablet computing device (46a).

12. A patient monitoring system (500) for providing a user access to patient data, the system comprising:
one or more sensors that are responsive to physiological characteristics of a patient (50) of one or more patients;
a data management system (DMS) (60, 60') configured to retrieve the patient data from a plurality of remotely located facility systems (18, 20, 40);
a docking station (600); and
a tablet computing device (46a) in communication with the one or more sensors and comprising a computer-readable storage device having instructions stored thereon which, when executed by the tablet computing device (46a), cause the tablet computing device (46a) to perform operations comprising:
determining that the tablet computing device (46a) is docked in a docking station (600) of the patient monitoring system (500); and
in response to determining that the tablet computing device (46a) is docked in a docking station (600) of the patient monitoring system (500), automatically triggering execution, by the tablet computing device (46a), of a single application, which is a patient monitoring application, preventing execution of other applications, and disabling functionality of one or more buttons of the tablet computing device (46a);
receiving, from the DMS (60, 60'), the patient data comprising one or more signals associated with one or more patients, at least a first sub-set of the patient data being associated with the patient (50) and being provided from one or more sensors that are responsive to the physiological characteristics of the patient (50);
processing the patient data to generate one or more graphical representations reflective of the patient data; and
displaying the one or more graphical representations on a touchscreen display of the tablet computing device (46a).

## Patentansprüche

1. Computerimplementiertes Verfahren zum Bereitstellen von Zugang zu Patientendaten für einen Benutzer einer Tablet-Rechenvorrichtung (46a), ausgeführt unter Verwendung eines oder mehrerer Prozessoren, wobei das Verfahren Folgendes umfasst:
Bestimmen, dass die Tablet-Rechenvorrichtung (46a) an einer Dockingstation (600) eines Patientenüberwachungssystems (500) angedockt ist; und
als Reaktion auf Bestimmen, dass die Tablet-Rechenvorrichtung (46a) an einer Dockingstation (600) des Patientenüberwachungssystems (500) angedockt ist, automatisches Auslösen einer Ausführung, durch die Tablet-Rechenvorrichtung (46a), einer Einzelanwendung, die eine Patientenüberwachungsanwendung ist, die Ausführung anderer Anwendungen verhindert und Funktionalität von einer oder mehreren Tasten der Tablet-Rechenvorrichtung (46a) blockiert; Empfangen, durch die Tablet-Rechenvorrichtung (46a) von einem Datenmanagementsystem (DMS)(60, 60'), das ausgelegt ist zum Abrufen der Patientendaten von mehreren abgesetzt befindlichen Einrichtungssystemen (18, 20, 40), der Patientendaten, die ein oder mehrere mit einem oder mehreren Patienten (50) verknüpfte Signale umfassen, wobei mindestens eine erste Untermenge der Patientendaten mit dem Patienten (50) verknüpft ist und von einem oder mehreren Sensoren, die auf physiologische Charakteristika des Patienten (50) reagieren, geliefert wird;
Verarbeiten der Patientendaten zum Erzeugen einer oder mehrerer graphischer Darstellungen, die die Patientendaten wiedergeben; und
Anzeigen der einen oder der mehreren graphischen Darstellungen auf einer Berührungsbildschirmanzeige der Tablet-Rechenvorrichtung (46a).

2. Verfahren nach Anspruch 1, wobei das DMS (60, 60') Daten zwischen mehreren abgesetzten Vorrichtungen (12) und einem oder mehreren Informationssystemen (42) synchronisiert und überträgt.

3. Verfahren nach Anspruch 2, wobei das DMS (60, 60') Zusatzinformationen für Übertragung und Speicherung in dem Informationssystem (42) von den mehreren abgesetzten Vorrichtungen (12) verarbeitet und vorbereitet.

4. Verfahren nach Anspruch 1, wobei eine zweite Untermenge der Patientendaten von einem Informationssystem bereitgestellt wird, das mit einer Einrichtung verknüpft ist, innerhalb der der eine oder die mehreren Patienten (50) behandelt werden.

5. Verfahren nach Anspruch 1, wobei die eine oder die mehreren graphischen Darstellungen auf der ersten Untermenge von Patientendaten basierende Wellenformen umfassen.

6. Verfahren nach Anspruch 1, wobei die eine oder die mehreren graphischen Darstellungen mit physiologischen Charakteristika von mehreren Patienten (50) des einen oder der mehreren Patienten (50) verknüpfte Wellenformen umfassen.

7. Verfahren nach Anspruch 1, wobei die eine oder die mehreren Tasten eine Home-Taste und eine Schlaf-Aufwach-Taste umfassen.

8. Verfahren nach Anspruch 1, ferner Folgendes umfassend:
Empfangen einer Benutzereingabe, die Authentifikationsdaten umfasst;
Bestimmen, dass die Benutzereingabe einem authentifizierten Benutzer entspricht; und
als Reaktion, Verlassen des Einzelanwendungsmodus, so dass die Tablet-Rechenvorrichtung (46a) in der Lage ist, mehrere Computerprogrammanwendungen auszuführen.

9. Verfahren nach Anspruch 1, wobei die Dockingstation (600) ferner einen oder mehrere mit medizinischen Standardgeräten kompatible Ports umfasst.

10. Verfahren nach Anspruch 1, wobei die Dockingstation (600) einen Einschubschlitz umfasst, der ausgelegt ist zum Aufnehmen der Tablet-Rechenvorrichtung (46a).

11. Verfahren nach Anspruch 1, wobei die Dockingstation (600) einen Verriegelungsmechanismus umfasst, der dafür ausgelegt ist, Entfernung der Tablet-Rechenvorrichtung (46a) zu verhindern.

12. Patientenüberwachungssystem (500) zum Bereitstellen von Zugang zu Patientendaten für einen Benutzer, wobei das System Folgendes umfasst:
einen oder mehrere Sensoren, die auf physiologische Charakteristika eines Patienten (50) aus einem oder mehreren Patienten reagieren;
ein Datenmanagementsystem (DMS)(60, 60'), das ausgelegt ist zum Abrufen der Patientendaten von mehreren abgesetzt befindlichen Einrichtungssystemen (18, 20, 40) ;
eine Dockingstation (600); und
eine Tablet-Rechenvorrichtung (46a) in Kommunikation mit dem einen oder den mehreren Sensoren und umfassend eine computerlesbare Speicherungsvorrichtung, die darauf gespeicherte Anweisungen aufweist, die bei Ausführung durch die Tablet-Rechenvorrichtung (46a) die Tablet-Rechenvorrichtung (46a) zum Durchführen von Operationen veranlassen, die Folgendes umfassen:
Bestimmen, dass die Tablet-Rechenvorrichtung (46a) an einer Dockingstation (600) des Patientenüberwachungssystems (500) angedockt ist; und
als Reaktion auf Bestimmen, dass die Tablet-Rechenvorrichtung (46a) an einer Dockingstation (600) des Patientenüberwachungssystems (500) angedockt ist, automatisches Auslösen einer Ausführung, durch die Tablet-Rechenvorrichtung (46a), einer Einzelanwendung, die eine Patientenüberwachungsanwendung ist, die Ausführung anderer Anwendungen verhindert und Funktionalität von einer oder mehreren Tasten der Tablet-Rechenvorrichtung (46a) blockiert;
Empfangen, von dem (DMS) (60, 60'), der Patientendaten, die ein oder mehrere mit einem oder mehreren Patienten verknüpfte Signale umfassen, wobei mindestens eine erste Untermenge der Patientendaten mit dem Patienten (50) verknüpft ist und von einem oder mehreren Sensoren, die auf die physiologischen Charakteristika des Patienten (50) reagieren, geliefert wird;
Verarbeiten der Patientendaten zum Erzeugen einer oder mehrerer graphischer Darstellungen, die die Patientendaten wiedergeben; und
Anzeigen der einen oder der mehreren graphischen Darstellungen auf einer Berührungsbildschirmanzeige der Tablet-Rechenvorrichtung (46a).

## Revendications

1. Procédé implémenté par ordinateur pour fournir à un utilisateur d'une tablette numérique (46a) un accès à des données patient, exécutées au moyen d'un ou de plusieurs processeurs, le procédé comprenant :
la détermination établissant que la tablette numérique (46a) est ancrée dans une station d'accueil (600) d'un système de surveillance de patient (500) ; et
en réponse à une détermination établissant que la tablette numérique (46a) est ancrée dans une station d'accueil (600) du système de surveillance de patient (500), le déclenchement automatique de l'exécution, par la tablette numérique (46a), d'une seule application, qui est une application de surveillance de patient, empêchant l'exécution d'autres applications, et désactivant une fonctionnalité d'un ou de plusieurs boutons de la tablette numérique (46a) ; la réception, par la tablette numérique (46a) en provenance d'un système de gestion de données (DMS) (60, 60') configuré pour récupérer les données patient dans de multiples systèmes d'installations à distance (18, 20, 40), des données patient comprenant un ou plusieurs signaux associés à un ou plusieurs patients (50), au moins un premier sous-ensemble des données patient étant associé au patient (50) et étant fourni depuis un ou plusieurs capteurs qui sont sensibles aux caractéristiques physiologiques du patient (50) ;
le traitement des données patient pour générer une ou plusieurs représentations graphiques reflétant les données patient ; et
l'affichage de la ou des représentations graphiques sur un écran tactile de la tablette numérique (46a).

2. Procédé selon la revendication 1, dans lequel le DMS (60, 60') synchronise et transfère des données entre de multiples dispositifs distants (12), et un ou plusieurs systèmes d'informations (42).

3. Procédé selon la revendication 2, dans lequel le DMS (60, 60') traite et prépare des informations auxiliaires pour un transfert vers le système d'informations 42 et une mémorisation dans celui-ci depuis les multiples dispositifs distants (12).

4. Procédé selon la revendication 1, dans lequel un second sous-ensemble des données patient est fourni depuis un système d'informations associé à une installation à l'intérieur de laquelle le ou les patients (50) sont traités.

5. Procédé selon la revendication 1, dans lequel la ou les représentations graphiques comprennent des formes d'onde basées sur le premier sous-ensemble de données patient.

6. Procédé selon la revendication 1, dans lequel la ou les représentations graphiques comprennent des formes d'onde associées à des caractéristiques physiologiques d'une pluralité de patients (50) du ou des patients (50).

7. Procédé selon la revendication 1, dans lequel le ou les boutons comprennent un bouton accueil et un bouton veille/sortie de veille.

8. Procédé selon la revendication 1, comprenant en outre :
la réception d'une entrée utilisateur comprenant des données d'authentification ;
la détermination que l'entrée utilisateur correspond à un utilisateur authentifié ; et
en réponse, la sortie du mode d'application unique de sorte que la tablette numérique (46a) puisse exécuter de multiples applications de programmes informatiques.

9. Procédé selon la revendication 1, dans lequel la station d'accueil (600) comprend en outre un ou plusieurs ports compatibles avec des dispositifs médicaux standards.

10. Procédé selon la revendication 1, dans lequel la station d'accueil (600) comprend un emplacement qui est configuré pour recevoir la tablette numérique (46a).

11. Procédé selon la revendication 1, dans lequel la station d'accueil (600) comprend un mécanisme de verrouillage qui est configuré pour empêcher de retirer la tablette numérique (46a).

12. Système de surveillance de patient (500) pour fournir à un utilisateur un accès à des données patient, le système comprenant :
un ou plusieurs capteurs qui sont sensibles aux caractéristiques physiologiques d'un patient (50) parmi un ou plusieurs patients ;
un système de gestion de données (DMS) (60, 60') configuré pour récupérer les données patient dans de multiples systèmes d'installations à distance (18, 20, 40) ;
une station d'accueil (600) ; et
une tablette numérique (46a) en communication avec le ou les capteurs et comprenant un dispositif de mémoire lisible par ordinateur sur lequel sont mémorisées des instructions qui, lorsqu'elles sont exécutées par la tablette numérique (46a), amènent la tablette (46a) à exécuter des opérations comprenant :
la détermination établissant que la tablette numérique (46a) est ancrée dans une station d'accueil (600) du système de surveillance de patient (500) ; et
en réponse à une détermination établissant que la tablette numérique (46a) est ancrée dans une station d'accueil (600) du système de surveillance de patient (500), le déclenchement automatique de l'exécution, par la tablette numérique (46a), d'une seule application, qui est une application de surveillance de patient, empêchant l'exécution d'autres applications, et désactivant une fonctionnalité d'un ou de plusieurs boutons de la tablette numérique (46a) ;
la réception, en provenance du DMS (60, 60'), des données patient comprenant un ou plusieurs signaux associés à un ou plusieurs patients, au moins un premier sous-ensemble des données patient étant associé au patient (50) et étant fourni depuis un ou plusieurs capteurs qui sont sensibles aux caractéristiques physiologiques du patient (50) ;
le traitement des données patient pour générer une ou plusieurs représentations graphiques reflétant les données patient ; et
l'affichage de la ou des représentations graphiques sur un écran tactile de la tablette numérique (46a).
